# FASCICULE DE BREVET EUROPEEN

(11) **EP 0 702 938 B1**
(45) Date de publication et mention de la délivrance du brevet: **10.05.2000**
(21) Numéro de dépôt: 95402125.9
(22) Date de dépôt: 21.09.1995
(51) Int. Cl.: A61B 19/08, A61F 13/44

(54) **Compresse ou champ opératoire à usage unique**
Chirurgisches Abdecktuch oder Kompresse für einmalige Verwendung
Single use surgical drape or compress

(30) Priorité: 23.09.1994 FR 9411357
(43) Date de publication de la demande: 27.03.1996
(73) Titulaire: Laboratoire Hydrex (SA), 69550 Amplepuis (FR)
(72) Inventeur: Carion, Jean-Pierre, F-59850 Nieppe (FR)
(74) Mandataire: Lachat, Salomé

(56) Documents cités:
- EP-A- 0 006 647
- FR-A- 2 352 664
- GB-A- 832 709
- GB-A- 1 393 239
- GB-A- 2 145 661
- US-A- 4 626 251

## Description

La présente invention est relative à une compresse ou un champ opératoire à usage unique utilisé notamment comme champ de paroi recouvrant les bords de la plaie après l'incision et avant la pose des écarteurs, et, à son procédé de fabrication. Ce type de champ peut aussi être utilisé comme champ abdominal pour recliner les viscères ou les anses intestinales. Lorsqu'il est préalablement humidifié, on peut l'utiliser pour protéger un organe fragile du dessèchement.

On connaît des champs opératoires en coton avec un grammage usuellement compris entre 140 et 200 g/m². Ces champs sont relavés et désinfectés après chaque usage, repliés, reconditionnés et restérilisés, d'où un risque de contamination accidentelle dans les blanchisseries lors des manipulations des champs opératoires maculés. Ces champs présentent encore l'inconvénient de donner naissance à des particules formées de bourres de coton susceptibles de provoquer des infections.

Pour pallier ces différents inconvénients inhérents aux champs connus à usages répétés après reconditionnement, la présente invention a pour objet un champ opératoire à usage unique constitué de nappes de voile non tissé hydrophile prises en sandwich entre des nappes de gaze hydrophile. Un champ opératoire conforme à l'invention comporte de préférence cinq couches, savoir trois nappes de gaze hydrophile, formant une pièce unique repliée pour obtenir la compresse, et deux nappes de voile non tissé hydrophile. Au moins trois bords du champ opératoire sont recouverts, à la façon d'un ourlet, par un ruban non tissé, replié sur les deux nappes extérieures de gaze hydrophile, et solidarisé à toutes les nappes qu'il enserre.

Le ruban non tissé utilisé pour former les ourlets sera de préférence en polypropylène qui facilite une soudure par ultrason. Un fil en polyester imprégné de sulfate de baryum, donc opaque aux rayons X, est pris dans au moins l'un des ourlets. Le fil en polyester est soudé par une soudure par ultrason à au moins l'un des autres composants (nappes, ruban) dudit champ opératoire. Une soudure par ultrason traverse les nappes et les deux branches du ruban formant ourlet. Des soudures par ultrason s'étendent sur toute la longueur du champ et sont réparties transversalement à des intervalles réguliers, sur toute la surface dudit champ.

Le champ opératoire décrit ci-dessus, conforme à l'invention, est fabriqué en continu selon le procédé exposé aux revendications 7 et 8.

Contrairement aux compresses ou champs opératoires hétérogènes connus, la compresse conforme à l'invention est fabriquée par pliage; elle ne présente aucun complexage, et plus particulièrement elle est obtenue sans utilisation d'un adhésif pour l'assemblage des couches.

L'utilisation de champs opératoires conformes à l'invention présente l'avantage d'un contact sur la peau avec une fibre naturelle qui ne «peluche» pas et de la capacité d'absorption d'une fibre non tissée composée de viscose absorbante et de polypropylène pour assurer la solidité du produit. Le choix entre ces différentes fibres est déterminé par leur coût et le pouvoir absorbant que l'on souhaite conférer au champ opératoire. Il est aussi possible d'utiliser en tant que non tissé d'autres matériaux qu'un polypropylène. Ce seront, par exemple, un polyester ou un polyamide. Ces fibres artificielles sont fusibles par ultrason.

En effet, l'association de matériaux différents, savoir un matériau tissé et un matériau non tissé, permet de cumuler les avantages respectifs des deux : le matériau tissé présente une résistance identique à l'état sec et à l'état mouillé, alors que le matériau non tissé, mouillé, a une résistance plus faible. Par contre, le matériau non tissé présente une très grande capacité d'absorption. Ainsi un champ opératoire conforme à la présente invention a un pouvoir d'absorption deux fois plus grand qu'un champ opératoire classique en matériau tissé tout en présentant une grande résistance.

Un champ opératoire composé de plusieurs nappes et fabriqué conformément à l'invention conduit à un rapport «souplesse / absorption» très supérieur à celui envisageable lors de l'utilisation d'un seul matériau. Ainsi, l'utilisation de matériaux tissés et non tissés selon l'enseignement de l'invention confère au produit fini les avantages de chacun des deux matériaux sans en conserver les inconvénients.

L'invention sera explicitée de façon purement indicative à l'aide des dessins annexés à titre d'exemples non limitatifs.

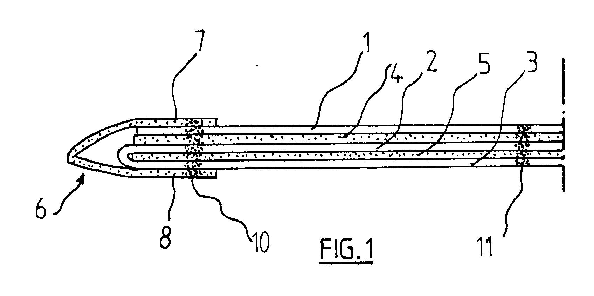

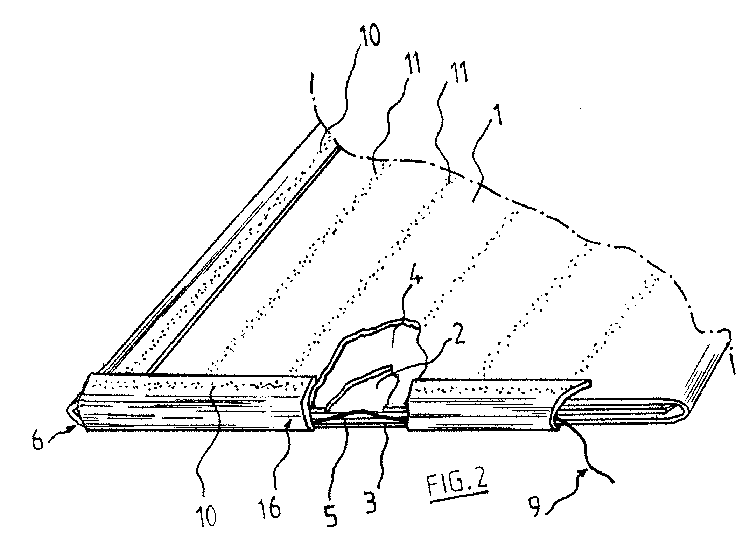

Les deux figures présentent pour la figure 1 une coupe d'un champ opératoire conforme à l'invention composé de cinq nappes superposées et pour la figure 2 une vue partielle en perspective cavalière et en écorché.

A la figure 1, on peut voir en coupe partielle les nappes (1), (2) et (3) qui sont en gaze hydrophile présentant un grammage généralement compris entre 15 à 30 g/m², de préférence 23 g/m². Comme on peut le voir à la figure 1, les nappes de gaz hydrophile (1), (2) et (3) constituent une pièce unique repliée. Les nappes intercalaires (4) et (5) sont en mélange de viscose et de polypropylène non tissé de grammage compris entre 20 et 30 g/m². Un ruban (6) en polypropylène enserre les cinq nappes (1), (2), (3), (4) et (5) entre ses deux branches (7) et (8). Une soudure (10) par ultrason traverse les cinq nappes (1), (2), (3) (4) et (5) et les deux branches du ruban (6). Les cinq nappes du champ sont traversées par des soudures (11) longitudinales (trame) distantes les unes des autres selon un pas de 5 à 8 cm afin de maintenir les cinq nappes assemblées en un complexe unique et d'éviter qu'elles ne soient séparées les unes des autres, risquant ainsi une détérioration du champ.

A la figure 2 qui reprend les mêmes éléments de la figure 1, on aperçoit les cinq nappes du champ qui sont traversées par des soudures (11) distantes les unes des autres selon un pas de 5 à 8 cm afin de maintenir lesdites nappes assemblées en un complexe unique et d'éviter qu'elles ne soient séparées les unes des autres, risquant ainsi une détérioration du champ. Les soudures peuvent être droites ou ondulées, ou encore en zigzag sous réserve d'utiliser un cylindre gravé lors de l'opération de soudure.

Comme montré plus particulièrement à la figure 2, une soudure (10) par ultrason traverse les cinq nappes (1), (2), (3) (4) et (5) et les deux branches (7) et (8) du ruban (16) disposé transversalement (chaîne). Un fil (9) en polyester court à l'intérieur du ruban, le long des chants de nappes. Ce fil (9) est transversal (chaîne) par rapport aux soudures longitudinales (11). Il est imprégné de sulfate de baryum pour être opaque aux rayons X. Le fil (9) est soudé en un point, généralement au milieu de sa longueur, par une soudure par ultrason à au moins l'un des autres composants (1), (2), (3), (4), (5), (6) dudit champ opératoire. Il ne peut donc s'échapper.

Un champ opératoire comportant cinq épaisseurs et conforme à l'invention s'obtient en plusieurs phases lors d'une fabrication en continu.
- *Dépôt*. Sur une nappe de gaze hydrophile de 1,60 m de laize, on dépose parallèlement, sans recouvrement, sur un côté de ladite nappe, deux nappes de voile non tissé hydrophile, de 0,53 m de laize ou une seule laize de 1,06 m de manière à laisser non recouverte environ un tiers de la largeur de ladite nappe de gaze hydrophile. Selon l'usage en chirurgie, la nappe de gaze hydrophile est de préférence de couleur verte ou bleue afin d'éviter tout éblouissement. La largeur des nappes est donnée à titre d'exemple. Il est bien entendu possible de travailler avec des nappes de largeur différente pour s'adapter aux usages locaux.
- *Dossage*. On effectue un premier dossage d'une partie de la nappe de gaze hydrophile sur l'une des nappes de voile non tissé par pliage de la partie laissée libre de la nappe de gaze hydrophile. Un second dossage par pliage conduit à une laize de 0,53 m, formée de 5 nappes de voile. On obtient une bande multicouche composée d'une nappe de gaze hydrophile, d'une nappe de voile non tissé, d'une nappe de gaze hydrophile, d'une nappe de voile non tissé, d'une nappe de gaze hydrophile. Les nappes extérieures de la bande multicouche sont en gaze hydrophile.
- *Pose d'au moins un ourlet longitudinal*. Au moins l'un des bords longitudinaux de la bande multicouche est recouvert d'un ruban en non tissé, formant ourlet, replié sur les deux nappes extérieures en gaze hydrophile et solidarisé aux nappes qu'il enserre. On remarque que dans le cas d'une bande multicouche à trois ou cinq nappes, il est possible de se limiter à un seul ourlet longitudinal.
- *Soudure longitudinale du ou des rubans formant ourlet et de la bande multicouche.* Le ou les rubans longitudinaux sont fixé par une soudure par ultrason et la bande multicouche est pourvue de soudures par ultrason distantes les unes des autres selon un pas régulier de 5 à 8 cm dans le cas de soudures droites traversant toutes ses nappes.
- *Découpe transversale à longueur de la bande multicouche*. On découpe la bande multicouche à la longueur voulue pour les champs opératoires individuels, en général 50 cm.
- *Soudure des ourlets transversaux*. On procède à la soudure des deux ourlets transversaux. Dans l'un de ces ourlets est intégré un fil en polyester imprégné de sulfate de baryum.

## Revendications

1. Compresse ou champ opératoire à usage unique caractérisé en ce que il est constitué de plusieurs nappes superposées (1 à 5), alternativement en gaze hydrophile (1), (2), (3) et en voile non tissé (4), (5) hydrophile, les nappes extérieures (1), (3) étant en gaze hydrophile et toutes les nappes en gaz hydrophile (1), (2), et (3) constituant une pièce unique repliée.

2. Compresse ou champ opératoire selon la revendication 1 caractérisé en ce qu' au moins trois de ses bords sont recouverts d'un ruban (6), non tissé, formant ourlet, replié sur les deux nappes extérieures (1), (3) de gaze hydrophile, et solidarisé à toutes les nappes qu'il enserre.

3. Compresse ou champ opératoire selon les revendications 1 et 2 caractérisé en ce que les nappes de voile non tissé (4), (5), disposées entre les nappes de gaze hydrophile (1), (2), (3), et le ruban (6) non tissé recevant les bords dudit champ opératoire contiennent une fibre synthétique fusible par ultrason comme du polyester, polyéthylène, ou polypropylène .

4. Compresse ou champ opératoire selon les revendications 2 et 3 caractérisé en ce qu'un fil (9) en polyester imprégné de sulfate de baryum, donc opaque aux rayons X, est pris dans au moins un ourlet.

5. Compresse ou champ opératoire selon la revendication 4 caractérisé en ce que le fil (9) est soudé par une soudure par l'ultrason à au moins l'un des autres composants (1) à (6) dudit champ opératoire.

6. Compresse ou champ opératoire selon les revendications 1 à 5 caractérisé en ce que des soudures longitudinales par ultrason (11), traversant toutes les nappes (1) à (5) du champ opératoire, s'étendent sur toute la longueur du champ et sont réparties transversalement à intervalles réguliers sur toute la surface dudit champ.

7. Procédé de fabrication en continu d'un champ opératoire conforme aux revendications précédentes caractérisé par les étapes suivantes
- dépôt sur une nappe de gaze hydrophile, sans recouvrement et parallèlement l'une à l'autre, d'au moins une nappe de non tissé, de préférence de deux nappes de non tissé dont la largeur de chacune correspond à un tiers de la largeur de la nappe de gaze hydrophile de manière à laisser libre un tiers de la largeur de cette dernière,
- dossage de la nappe de gaze hydrophile de manière à ce que la gaze hydrophile forme les couches extérieures d'une bande multicouche,
- pose du ou des rubans latéraux formant les ourlets longitudinaux,
- soudures longitudinales par ultrason embrassant toutes les couches du ou des rubans formant les ourlets longitudinaux et, à intervalles réguliers répartis sur toute la largeur de la bande multicouche, de ladite bande multicouche,
- découpe transversale de la bande multicouche à longueur du champ opératoire individuel,
- soudure par ultrason des rubans formant les ourlets transversaux non tissés autour des découpes transversales de la bande multicouche.

8. Procédé de fabrication d'un champ opératoire selon la revendication 7 caractérisé en ce que un fil en polyester (9) imprégné de sulfate de baryum est posé le long d'au moins l'une des découpes transversales du champ opératoire avant la soudure par ultrason des rubans non tissés formant les ourlets transversaux desdites découpes transversales afin d'intégrer ledit fil dans ledit ourlet.

## Patentansprüche

1. Chirurgisches Abdecktuch oder Kompresse für einmalige Verwendung **dadurch gekennzeichnet,** daß es mehrere übereinander angeordnete Schichten (1 bis 5) abwechselnd aus saugfähiger Gaze (1), (2), (3) und saugfähigem Faservlies (Nonwowen) (4), (5) umfaßt, wobei die äußeren Schichten (1), (3) aus saugfähiger Gaze sind und alle Schichten aus saugfähiger Gaze (1), (2), (3) aus einem einzigen gefalteten Stoffstück bestehen.

2. Chirurgisches Abdecktuch oder Kompresse nach Anspruch 1 **dadurch gekennzeichnet,** daß mindestens drei seiner Ränder mit einem Band (6) aus Faservlies umfaßt sind, wobei das Band (6) einen die Ränder umgreifenden Saum bildet, der auf den äußeren Schichten (1), (3) aus saugfähiger Gaze aufliegt und mit allen umgriffenen Schichten fest verbunden ist.

3. Chirurgisches Abdecktuch oder Kompresse nach Anspruch 1 und 2 **dadurch gekennzeichnet**, daß zwischen den Schichten (1), (2), (3) aus saugfähiger Gaze angeordneten die Schichten (4), (5) aus Faservlies und das die Ränder umgreifende Saumband (6) aus Faservlies eine bei Ultraschall schmelzbare Kunststofffaser enthalten, z.B. aus Polyester, Polyethylen, Polyprpylen.

4. Chirurgisches Abdecktuch oder Kompresse nach Anspruch 2 und 3 **dadurch gekennzeichnet,** daß ein mit Bariumsulfat getränkter, für Röntgenstrahlen undurchläßiger Polyesterfaden (9) wenigstens in eines die Ränder umfassenden Saumbänder eingefügt ist.

5. Chirurgisches Abdecktuch oder Kompresse nach Anspruch 4 **dadurch gekennzeichnet,** daß der Faden (9) mittels Ultraschallschweissung mit wenigstens einem der anderen Bestandteile (1 bis 6) des Abdecktuchs verbunden ist.

6. Chirurgisches Abdecktuch oder Kompresse nach Anspruch 1 bis 5 **gekennzeichnet durch** längsgerichtete, durch Ultraschallschweissung angebrachte, alle Schichten (1 bis 5) des Abdecktuchs durchsetzende Längsschweißnähte, welche sich über die gesamte Länge des Abdecktuchs erstrecken und in regelmäßigen Abständen voneinander über die gesamte Breite des Tuchs verteilt angeordnet sind.

7. Kontinuierliches Herstellungsverfahren von einem chirurgischen Abdecktuch oder einer Kompresse nach einem der vorangehenden Ansprüche 1 bis 6, gekennzeichnet durch die folgenden Verfahrensschritte :
- Auflegen auf ein Stück saugfähiger Gaze von wenigstens einem, vorzugsweise von zwei Faservliesstücken, deren Breite je einem Drittel der gesamten Breite des Gazestücks entspricht, wobei die Faservliesstücke parallel und ohne gegenseitige Überdeckung nebeneinander angeordnet sind, so daß ein Drittel des Gazestücks freigehalten wird,
- Falten des Stücks saugfähiger Gaze, so daß diese die äußeren Schichten eines vielschichtigen Bandes bildet,
- Anbringen entlang der Längsränder des vielschichtigen Bandes von Seitensaumbändern aus Faservlies,
- Ultraschallschweissung der Längsschweißnähte, welche alle Schichten des Abdecktuchs und die die Längsränder umgreifenden Saumbänder durchsetzen, wobei die Längsschweißnähte in regelmäßigen Abständen zueinander über die gesamte Breite des vielschichtigen Bandes verteilt angeordnet sind,
- quergerichtetes Ablängen der einzelnen Abdecktücher vom vielschichtigen Band,
- Anbringen der die Querränder umgreifenden Saumbänder und Befestigung durch Ultraschallschweissung.

8. Herstellungsverfahren von einem chirurgischen Abdecktuch oder einer Kompresse nach Anspruch 7 dadurch gekennzeichnet, daß vor der Befestigung der die Querränder des Abdecktuchs umgreifenden Saumbänder ein mit Bariumsulfat getränkter Polyesterfaden (9) in wenigstens einen der Quersäume eingelegt wird.

## Claims

1. A single-use compress or drape characterized in that it is constituted by a plurality of superposed sheets (1 to 5) alternately of hydrophilic gauze (1, 2, 3) and of hydrophilic non-woven web (4, 5), the outer sheets (1, 3) being of hydrophilic gauze and all of the hydrophilic gauze sheets (1, 2, and 3) constituting a single folded piece.

2. A compress or drape according to claim 1, characterized in that at least three of its edges are covered in a non-woven tape (6) forming a hem, folded over the two outer sheets (1, 3) of hydrophilic gauze, and secured to all of the sheets that it encloses.

3. A compress or drape according to claims 1 and 2, characterized in that the sheets of non-woven web (4, 5) disposed between the sheets of hydrophilic gauze (1, 2, and 3), and the non-woven tape (6) receiving the edges of said drape contain a synthetic fiber that can be melted by ultrasound, such as polyester, polyurethane, or polypropylene.

4. A compress or drape according to claims 2 and 3, characterized in that a thread (9) of polyester that is impregnated with barium sulfate, and that is therefore opaque to X-rays, is included at least in the hem.

5. A compress or drape according to claim 4, characterized in that the thread (9) is bonded by ultrasound bonding to at least one of the other components (1 to 6) of said drape.

6. A compress or drape according to claims 1 to 5, characterized in that longitudinal ultrasound bonds (11) passing through all of the sheets (1 to 5) of the drape extend over the full length of the drape and are distributed transversely at regular intervals over the entire area of said drape.

7. A method of continuously manufacturing a drape according to the preceding claims, the method being characterized by the following steps:
· on a sheet of hydrophilic gauze, there are deposited in parallel and without overlap at least one sheet of non-woven cloth, and preferably two sheets of non-woven cloth each being of a width that corresponds to one-third the width of the sheet of hydrophilic gauze so as to leave unoccupied one-third of the width thereof;
· the sheet of hydrophilic gauze is folded in such a manner that the hydrophilic gauze forms the outer layers of a multilayer strip;
· the lateral tape(s) forming the longitudinal hems are put into place;
· ultrasonic longitudinal bonding is performed of said multilayer strip through all of the layers of the tape(s) forming the longitudinal hems and, at regular intervals distributed across the entire width of the multilayer strip;
· the multilayer strip is cut transversely to length to form individual drapes; and
· tapes forming non-woven transverse hems are ultrasonically bonded around the transverse cuts through the multilayer strip.

8. A method of manufacturing a drape according to claim 7, characterized in that a polyester thread (9) impregnated with barium sulfate is placed along at least one of the transverse cuts of the drape prior to the ultrasonic bonding of the non-woven tapes forming the transverse hems of said transverse cuts so as to integrate said thread in said hem.
